Europäisches Patentamt

European Patent Office

Office européen des brevets

(11) Publication number: **0 241 443**
**B1**

(12) **EUROPEAN PATENT SPECIFICATION**

(45) Date of publication of patent specification: **08.08.90**

(51) Int. Cl.[5]: **G 01 N 33/564**

(21) Application number: **87850115.4**

(22) Date of filing: **08.04.87**

(54) Reagent for and procedure in the determination of C3a by immunochemical assay.

(30) Priority: **11.04.86 SE 8601643**

(43) Date of publication of application:
**14.10.87 Bulletin 87/42**

(45) Publication of the grant of the patent:
**08.08.90 Bulletin 90/32**

(84) Designated Contracting States:
**AT BE CH DE ES FR GB GR IT LI LU NL SE**

(56) References cited:
**EP-A-0 097 440**

**Scand. J. Immunol., Vol. 22, 1984, pages 703-710
; B. NILSON et al.: "An Assessment of the
Extence of Antigenic Anology between
Physiological Bound C3 and C3 Denarurated by
Sodium Dodecyl Sulphate"**

(73) Proprietor: **Nilsson, Ulf Ragnar
Storgatan 18 C
S-753 31 Uppsala (SE)**
(73) Proprietor: **Nilsson, Sven Ivar Bo
Byggmästargatan 10D
S-754 35 Uppsala (SE)**
(73) Proprietor: **Svensson, Karl-Erik
Västertorg 5A
S-752 43 Uppsala (SE)**

(72) Inventor: **Nilsson, Ulf Ragnar
Storgatan 18 C
S-753 31 Uppsala (SE)**
Inventor: **Nilsson, Sven Ivar Bo
Byggmästargatan 10D
S-754 35 Uppsala (SE)**
Inventor: **Svensson, Karl-Erik
Västertorg 5A
S-752 43 Uppsala (SE)**

(74) Representative: **Kummelsten, Per-Arne et al
UPPSALA PATENTBYRA P.O. Box 9013
S-750 09 Uppsala (SE)**

Courier Press, Leamington Spa, England.

# EP 0 241 443 B1

**Description**

The invention is concerned with the determination of a substance, C3a, which is released upon activation of the mammalian complement system (= C). In particular human C3 and human C3a are concerned.

The complement system consists of about 20 components which have to react in a well-defined reaction sequence (= complement activation) in order that destruction of e.g. invading microorganisms can take place in the final stage. The complement system is considered to be a fundamental element of the mammal's defense against infections caused by bacteria and by viruses. The components discovered first were called "complement factors" and were named C1, C2, ... C9.

Activation may be induced by a large number of substances such as e.g. immune complexes, aggregated immunoglobulin, protein A and bacterial polysaccharides, and can be correlated to inter alia some autoimmune diseases and inflammatory processes. It has been suggested before that the fragments released on activation should be utilized as markers to indicate that activation has taken place. Thus, a number of immunochemical methods have been developed for measuring C3a, C4a and C5a (3, 4, 16).

C3a, C4a and C5a taken together have been given one common name, "anaphylatoxins". In their circulating forms they are quickly deactivated by losing a terminal arginyl residue and are then called C3ai, C4ai and C5ai respectively. Their immunochemical assay utilizes antibody preparations reacting specifically with (i) the anaphylatoxin to be assayed for, (ii) the corresponding desArg form and (iii) other anaphylatoxin fragments having determinants which are the same as determinants on the said anaphylatoxin and against which the antibody preparation employed is directed. What is measured is thus the sum of the components carrying a certain type of determinants.

The term "immunochemical assay for C3a" in the context of the present invention refers to assaying for C3a, C3ai and such fragments of C3a that contain antigenic determinants against which the anti-C3a antibody preparation of this invention is directed.

An immunochemical method for quantitatively or qualitatively assaying for a complement factor or its fragments always relies on using an antibody preparation directed against the species assayed for. The preparation is allowed to react with a sample containing the factor assayed for, whereupon the immune complex that has formed is taken as an indication of the presence of the factor, or as a measure of its amount. If the species assayed for is a fragment (for example C3a) it is imperative that the corresponding original complement factor (C3) does not cross react with the said fragment in a disturbing manner. It has not been possible heretofore to produce antibody preparations of sufficient C3a specificity; for this reason the prior art methods have always involved a separation as a first step. All the commercially available tests for C3a exploit the fact that this fragment is highly stable against denaturation. In a strongly acidic medium it is therefore possible to precipitate C3 and to separate it from C3a before C3a is quantified immunochemically.

C3 is composed of two polypeptide chains called the alpha chain and the beta chain respectively. These are linked together by disulfide bonds. When complement is activated C3 is cleaved into fragments C3a (molecular weight 9 000 daltons = about 5% of C3) and C3b. The C3a fragment is believed not to participate in the subsequent activating sequence. C3b is necessary for continued activation, inasmuch as it is required for the conversion of C5 to C5b with release of C5a. In the course of the further degradation of C3b under physiological conditions, fragments C3bi, C3d,g and C3c and others are formed. C3a and C3d,g consist of portions of the alpha chain. C3c and C3b contain portions of both the alpha and beta chain. The positions of the fragments in the native C3 molecule can be represented schematically as follows:

There are several more S—S bridges, both inter-chain and intra-chain. C3b → C3bi + C3f; C3bi → C3c + C3d,g.

C3b is capable of covalently binding to a large number of substrates like for instance immune complexes and cell walls. When fragmentation occurs or when fragments are bound to substrates it has been shown that some antigenic determinants disappear whereas new ones are exposed in the fragments formed: So-called neoantigens are generated.

In a number of papers published earlier, the inventors have shown that the determinants brought forth upon binding to erythrocytes can be found also in denatured C3 (9, 11, 12, 13). On the basis of these findings the inventors have classified antigenic determinants of C3 as being (a) C3(S) which are stable determinants present both in native C3 and in denatured C3, (b) Ce(D), these being determinants forming upon denaturation, and (c) C3(N), these being native determinants that are replaced by C3(D) upon denaturation. Examples of denaturing media apt to generate C3(D) determinants are SDS (sodium dodecyl sulfate = sodium lauryl sulfate), guanidine hydrochloride, acidic and alkaline solutions (pH > 10, pH < 2,8) and deoxycholates. Results published up to now have demonstrated the existence of C3(D) determinants only in the C3b region of denatured C3. The present invention is based on the finding that denaturation of C3 may bring forth novel, unique C3(D)-type determinants in the C3a region of C3. These determinants are to be found also in circulating forms of C3a where they have been generated due to the formation of C3a from C3. The basis of the invention thus are newly discovered neoantigens.

One object of the invention is to provide anti-C3a antibody preparations which are directed against neoantigens. A second object of the invention is to provide improved methods for immunochemical assays for C3a (quicker, more precise and more specific than earlier methods). A third object is to provide a C3a assay in which the immunochemical reaction between C3a and its homologous antibody preparation may be carried out in the presence of native C3.

The antibody preparation of this invention is directed against at least one determinant which is common to C3a and denatured C3 but is not accessible in native C3. Thus the cross reactivity of C3a and C3 to the antibody preparation of the invention has to be such that the preparation can be used for demonstrating clinically significant levels of C3a in C3-containing samples. In its reaction with C3a it is substantially not inhibited by native C3. For this reason it can be used for immunochemically determining C3a in the very presence of native C3, for example in plasma samples where the clinically relevant measuring range is considered to be 40 to 1 000 ng per liter.

Further characteristics of the invention are set forth in the attached claims forming an integral part of this specification.

The antibody preparation of the invention has the specificity as stated above. It may be in the form of a monoclonal antibody or in the form of an antiserum (polyclonal).

Antibodies contained in the preparation may be fragmentized and/or derivatives may be formed, the important point being that the fragments and/or derivatives possess a biospecific immune-type affinity of the aforesaid specificity and cross reactivity. The preparation may be in the form of a solution with various additions of such chemicals as are required for any particular use contemplated. What matters here is that the solution should not contain any disturbing amount of anti-C3a antibodies with which C3a and C3 will cross react. The anti-C3a antibody-active components of the preparation may be provided with analytically detectable groups or may be bound to phases that are insoluble in the test medium — so-called "solid" phases.

For producing an antibody preparation according to the invention, cells potentially capable of producing antibodies of the specificity as prescribed in conformity with the invention are caused to secrete such antibodies which are then isolated (and optionally further purified) so as to remove those anti-C3a antibodies that do not fulfill the specificity requirements. The process involves the raising of a humoral immune response, the antibodies of which being then selected with respect to their specificities for an antigenic determinant in C3a which is not accessible in the C3a region of native C3, in particular for a so-called C3(D) antigen. There are two main routes for the production — the polyclonal technique and the monoclonal technique.

In a vertebrate (e.g. mammal such as mouse or rat) secretion may take place *in vivo* as a result of immunization with an appropriate immunogen. The immune response thus obtained will give a polyclonal antibody preparation containing the desired antibody/antibodies in admixture with antibodies directed against other determinants on the immunogen. For removal of these latter antibodies recourse may be had to so-called immunosorbent purification (IS purification), for instance performed on solid-phase-bound forms of native or denatured C3 or C3a. Depending on the immunosorbent employed the antibodies concerned will be found either in the eluate or on the adsorbent. In the case of the antibodies contemplated in the present context, IS purification gives low yields and involves laborious procedures.

The best method for obtaining a good antibody preparation according to the invention is a so-called monoclonal technique (see for instance ref. 6) by which after immunization the plasma cells producing antibodies are fused with a myeloma cell, to thus become capable of quick and uninterrupted growth. By cloning and culturing the hybrid cells that produce the anti-C3a antibodies of this invention it is possible to obtain monoclonal anti-C3a antibody preparations which will in principle react only with the desired type of determinants on C3a. Cultivation of the selected cell clones for producing the antibody preparations of the invention may be carried out in cell cultures *in vitro* or as ascites tumors. Purification and isolation may be performed in the same manner as purification and isolation of any antibodies in general — by salt precipitation or by means of various chromatographic methods like for instance ion exchange, affinity and gel chromatography.

The immunogen employed in the immunization procedure must have the required determinants, by preference C3(D) determinants present exclusively in the C3a region of denatured C3. Denaturation should preferably have been carried out in an aqueous solution with an SDS concentration exceeding $10^{-3}$M (pH about 7, room temperature) but may also alternatively have been carried out with other agents creating the same type of exclusive C3a-determinants. What is important here is not the particular denaturating agent as such but that the denaturation has been performed in a manner such as to expose at least one antigenic determinant that is present in C3a but is not accessible in native C3. Having become aware that these antigenic determinants do exist one is enabled in principle to use also C3a as the immunogen, especially if this is employed in combination with a monoclonal technique.

The term "denaturation" above of course does not comprise any such irreversible changes that destroy the ability of the determinant to produce an immune response (hydrolysis might give such effects).

According to the immunochemical assay method of this invention, the antibody preparation is employed for immunochemically assaying for C3a. A large number of general and known per se immunochemical testing methods are available which can be used for this purpose and to which the invention is applicable. They comprise, in the case of C3a, reacting anti-C3a antibody-active components with a sample containing C3a to thus form an immune complex the formation and amount of which are a quantitative measure and qualitative measure respectively of the presence of C3a in the sample. Also other immune reactants or other reactants capable of biospecific affinity reactions with components of the immune complex may be added in order to facilitate quantification. An expedient commonly employed is to add a reactant provided with a marker group, a so-called "analytically detectable" group. Proportions of the reactants are chosen so that the amount of labeled reactant incorporated in the complex or remaining unincorporated will be a measure of the presence of C3a in the sample.

According to one classification system, the methods may be classed as either homogeneous or heterogeneous methods. Homogeneous methods assay for a labeled reactant *without* involving any physical separation of the labeled reactant incorporated in the complex from its non-incorporated form. When heterogeneous methods are employed the two forms of the labeled reactant are separated physically from each other before the labeled reactant is assayed for in either one or both of the two forms. For ease of separation it is helpful if one of the reactants is insoluble in the test medium.

According to a second classification system, the methods may be classed as being either competitive or noncompetitive methods. In a competitive method the arrangement is such that two immune reactants having a common epitope (determinant) are made to compete for an insufficient number of homologous binding sites on an immunological counterpart. As applied to C3a determinations, the C3a of the sample is made to compete with labeled C3a or a solid-phase-bound form thereof for anti-C3a antibodies according to the invention. The amount thus reacting with the antibodies is a measure of the content of C3a in the sample. In a noncompetitive method, the reactants are chosen such that no competition can occur.

According to a third classification system, the methods may be classed as being either precipitation or nonprecipitation methods. For the precipitation method as applied to the present invention, an anti-C3a antibody preparation is chosen such that its reaction with the C3a of the sample will give a precipitate, or such that the resultant immune complex is subsequently precipitated by addition of an excess of precipitating agent such as polyethylene glycol, anti-antiserum or solid-phase-bound anti-antibody. It goes without saying that in case a labeled reactant is employed the anti-antiserum or the solid-phase-bound anti-antibody chosen will be one that is not directed against the labeled reactant.

According to a fourth classification system, the methods are classed according to the marker group employed; the methods are thus radio-, enzyme, fluorescence, chemiluminescence, enzyme-substrate and immunochemical methods.

Among immunochemical methods may be mentioned also immunoelectrophoresis, particle agglutination, immunodiffusion, and microscopy with labeled antibodies.

Monoclonal antibodies directed against nonrepetitive determinants in an antigen cannot be used for certain types of precipitation methods.

The invention is applicable to C3a assays in various body fluids and tissues containing C3a. It has been known heretofore that C3a is present in blood, plasma, serum, urine, synovial fluid and cerebrospinal fluid.

Conditions employed for carrying out the immunochemical reaction(s) are such as are usual in this type of assay methods. For instance temperatures may be chosen within the range of 0—40°C, especially 15—40°C. A suitable pH lies within the range of 4,5—9, preferably about 5—8,6. Too high (> 10) and too low (< 3) pH values will give denaturation effects in C3 (so that C3(D) determinants are formed) and are to be avoided. Quite generally of course measures and steps have to be taken so as to avoid activation of complement or denaturation if a C3a assay is carried out in the presence of C3. Complement activation involves formation of additional C3a and requires the presence of proteases and divalent calcium and/or magnesium ions. It is therefore suitable to add protease inhibitors and/or to add agents that will form complexes with these ions, like EDTA. Detergents and buffer systems if added should be of a kind that will not have a denaturing effect on C3.

The invention will now be illustrated by means of a number of examples which form a part of this specification and are not to be construed as limiting the scope of the invention in any way.

## Materials and Methods

*C3 preparations:*

Native C3(C3(SN)) was purified as described previously (10). Denaturation of 100 μg of C3(C3(SD)) was performed in 100 μl of $2 \times 10^{-3}$M SDS for 30 min at 37°C. Denatured-reduced C3 and isolated C3 alpha and C3 beta chains (C3(D)) were prepared as in Nilsson et al (11). Elastase-generated C3a, C3c and C3d were a kind gift from Dr Brian Tack, Scripps Clinic and Research Foundation, La Jolla, USA (15). C3b was prepared in the presence of 1% (w/v) trypsin (TPCK treated, Worthington, USA) for 2 min at room temperature (10). C3bi was obtained by incubating C3b with 5 µg of factor H and 1 µg of factor I for 60 min at 37°C. Radiolabelling of native C3 with $^{125}$I was performed as described previously by a lactoperoxidase technique to a specific activity of 30 000 cpm/µg protein (9).

*Antibody preparations:*

Antibodies were raised against and selected for specificity for SDS-denatured C3. Two Balb/c female mice, 8—12 weeks of age, were primed with 24 µg of human SDS-denatured C3 in Freund's complete adjuvant (FCA, Behringwerke AG, West Germany) together with 20 µg of Lipopolysaccharide W (LPS, Difco, cat no 3120—25) subcutaneously. Eight weeks later, on day four before fusion, 100 µg of SDS-denatured C3 in phosphate buffered saline, pH 7,4, was given intraperitoneally (i.p.). Hybridomas were produced according to standard procedures (2, 5), with the following minor modifications. Four different Sp 2/0 lines were used. Original Sp 2/0.Ag 14 (14) growing in standard Dulbecco's Modified Eagle's Medium (DMEM, Paisley, Scotland, cat no 041—1966) containing 5% fetal calf serum (FCS, Gibco, cat no 011—6290) and 25 µg/ml of Gentamycin (cat no G—7 507, Sigma Chem Co, USA) was the progenitor line. Two different subclones selected for growth in serum-free medium and one subclone growing in low serum medium Hy-0.1 were also used. Standard DMEM containing 10% FCS or HY-0.1 media were used for selection and cloning. The clones were screened for binding to SDS-denatured C3 in the direct-binding ELISA. 14 clones were randomly selected and further tested.

Established hybridoma lines were expanded by fed-batch type culture in TC flasks to 200 ml vol. The monoclonal antibodies were purified utilizing cation exchange chromatography as described earlier (1).

*Enzyme-linked immuno sorbent assay (ELISA):*
*Direct-binding assay:*

Serially diluted anti-Ce antibodies were allowed to bind to constant amounts of C3 or C3 fragments adsorbed to microtitre plate wells. The bound antibodies were thereafter quantified by anti-rabbit or anti-mouse immunoglobulins conjugated with horseradish peroxidase (HRP) (DAKO Immunoglobulins A/S, Denmark). Phosphate buffered saline (PBS) containing 0.1% TWEEN 20 (v/v) and 0.1% (w/v) bovine serum albumine (BSA) was used as a working solution.

1. 200 µl each of C3, C3 alpha and beta chains, SDS-denatured C3, C3c and C3d in PBS (corresponding to 20 nmol of C3/1) was adsorbed to the plastic surface of the different wells of microtitre plates (Immunoplate II F, Nunc, Denmark) overnight at 4°C.
2. 100 µl of serially diluted antibody preparations was incubated with the wells of step 1 for 60 min at room temperature (RT).
3. 100 µl of swine anti-immunoglobulins conjugated with HRP was allowed to bind to surface-associated anti-C3 antibodies from step 2 for 60 min at RT.
4. The enzyme reaction was started by the addition of 100 µl of colour reagent (20 mg of 1,2-phenylenediamine-dihydrochloride (Fluka AG, Switzerland) and 10 µl 30% $H_2O_2$ in 75 ml of 0.1 M citrate/phosphate buffer pH 5,0). The reaction was stopped by 100 µl of 1 M $H_2SO_4$ after approximately 10 min. The stain was quantified spectrophotometrically at 492 nm.
Following steps 1—3, the wells were rinsed extensively 3 times in saline containing 0.1% TWEEN 20.

*Inhibition assay:*

This assay was a modification of the direct-binding assay. 100 µl of serially diluted sample was allowed to compete with the adsorbed antigen for binding to a constant amount of antibody. The dose of antibody was selected to give $OD_{492}$ = 1 when binding to preadsorbed native C3 for 60 min at RT as described in the direct-binding assay. Following these initial steps the inhibition assay was completed by performing steps 3—4 of the direct-binding assay.

1. 200 µl of C3a 40 pg/ml was preincubated for adsorption in each well of the microtitre plate whereupon the wells were emptied and rinsed.
2. 50 µl of sample (EDTA-plasma 1/20 or serial dilution of C3a for the standard curve) + 100 µl of monoclonal anti-C3a were (preincubated) and added to the wells of step 1.
The standard curve is a serial dilution of a stock solution of C3a 50 pg/ml. From the standard curve the plasma concentration is obtainable.

*Specificity of monoclonal anti-C3 antibodies for solid-phase C3 and C3 fragments in ELISA*

The selected monoclonal antibodies were tested by the direct-binding ELISA for binding to native C3, SDS-denatured C3, protease-generated C3 fragments (C3a, C3c and C3d) and C3 alpha and C3 beta chains.

| C3(SN) | C3(SD) | C3c | C3d | C3a | C3alpha | C3beta |
|--------|--------|-----|-----|-----|---------|--------|
| 14 | 14 | 10 | 2 | 1 | 11 | 0 |

The antibody specific for C3a is an anti-C3a antibody and was further studied.

5

*Fig 1*

Inhibition of monoclonal anti-C3a antibody to plastics-adsorbed C3a in inhibition ELISA with soluble purified native C3 (●), SDS-denatured C3 (□), native C3 in EDTA-plasma (△), and C3a (○).

Doses (by weight) of SDS-denatured C3, purified native C3, and native C3 in EDTA-plasma for obtaining an equivalent inhibition of binding as compared to C3a are larger by a factor of 27, 243 and > 10 000 respectively.

*Fig. 2*

Concentration of C3a in EDTA-plasma from normal individuals and from patients with C1q-binding immune complex and with coronary insufficiency (these latter having undergone bypass surgery while lying in a heart-lung machine).

*Fig. 3*

Correlation of C3a concentrations as measured according to the invention and as measured by means of a commercial test (UpJohn), r = 0.9.

*Fig. 4*

Shows how C3a conc. is affected by 1 to 10 freeze-thaw cycles. No significant influence on the binding can be observed.

*Fig. 5*

The anti-C3a antibody employed in the above-described ELISA is here employed in an inhibition RIA.

1. 50 µl of anti-C3a + 10 µl of $^{125}$I—C3a + 50 µl of sample are incubated for 30 min at 37°C.
2. 2 ml of anti-mouse Ig tethered to Sepharose (Pharmacia RIA-Sepharose) are added, and incubation continues at room temperature for 30 min.
3. $^{125}$I cpm is measured after centrifugation of particles and discarding of supernatant.

Following step 1—3 the wells were rinsed extensively.

In this figure, the binding of $^{125}$I—C3a to Sepharose is inhibited with C3a (●) and native C3 in EDTA-plasma, but the doses differ by a factor of at least 100.

REFERENCES

1. Carlsson, M et al (1985) J Immunol Meth 79: 89—98
2. Goding, JW (1980) J Immunol Meth 39: 285—308
3. Gorski, JP et al (1981) J Immunol Meth 47: 61—73
4. Hugli, T.E. et al (1980) In: Laboratory and Research Methods in Biology and Medicine, Eds Nakamura, R.M. et al (Alan R. Liss, New York) 4:443—60
5. Lindell, P et al (1986) Development of a new set of monoclonal antibodies against Neisseria gonorrhoeae (Manuscript in preparation)
6. Köhler et al (1975) 256: 495—7
7. Lachmann, PJ et al (1982) J Exp Med 156: 205—16
8. Laemmli, UK et al (1973) J Mol Biol 80: 575—99
9. Nilsson, B et al (1985) Scand J Immunol 22: 703—10
10. Nilsson, UR et al (1975) J Immunol 114: 815—22
11. Nilsson, UR et al (1980) Mol Immunol 17: 1319—33
12. Nilsson, UR et al (1982) J Immunol 129: 2594—97
13. Nilsson, UR et al (1982) Mol Immunol 19: 1391
14. Schulman, M et al (1978) Nature 276: 269—70
15. Tack, BF et al (1981) Meth Enzymol 80: 64—101
16. Wagner, JL et al (1984) Anal Biochem 136: 75—88

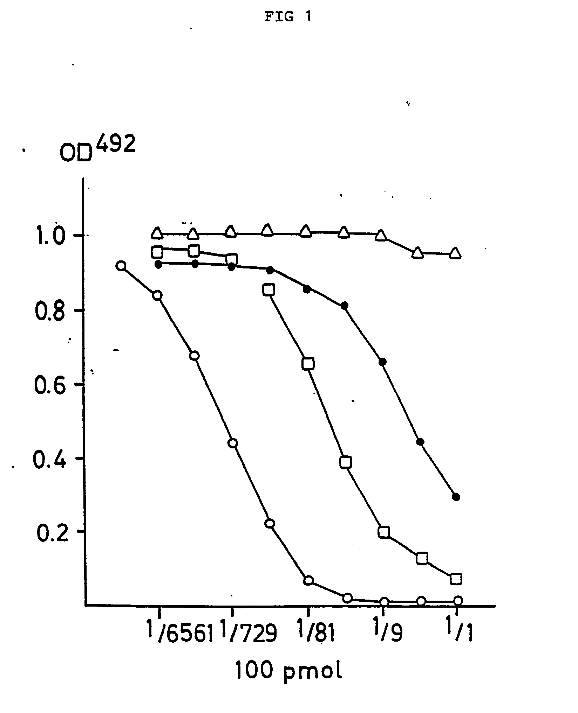

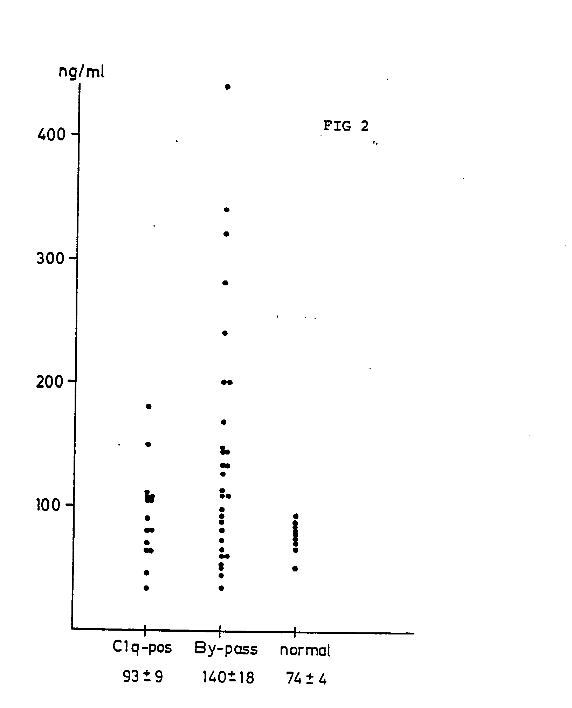

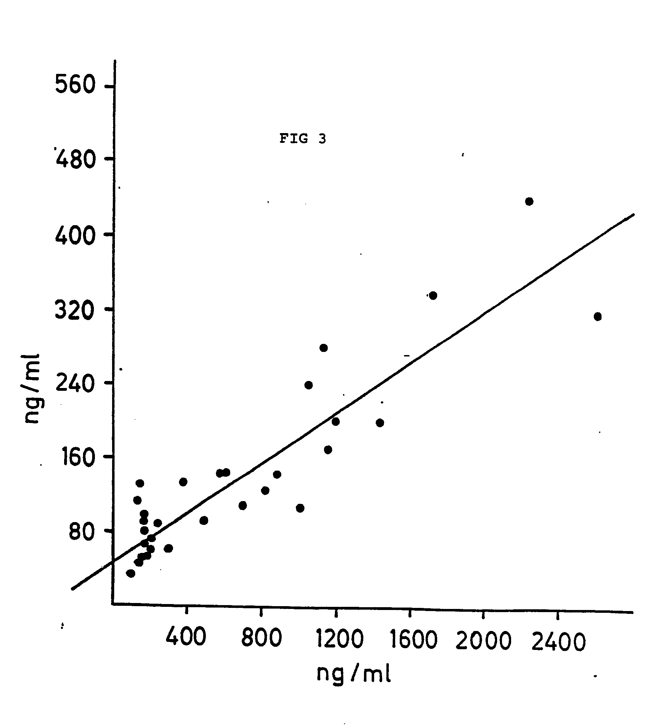

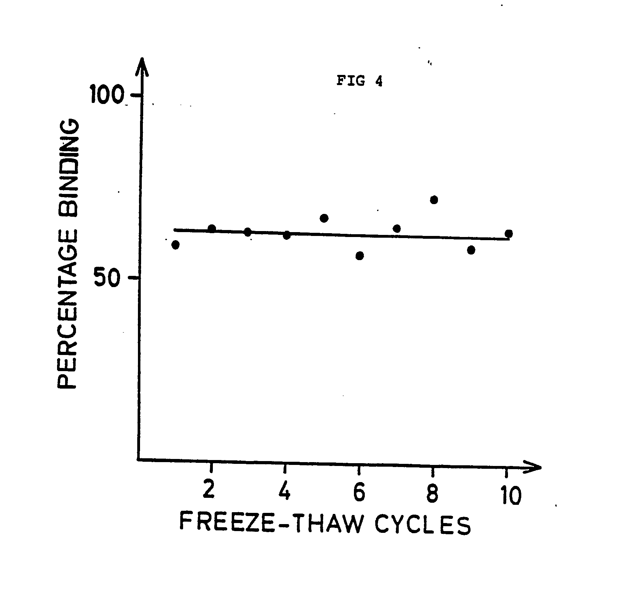

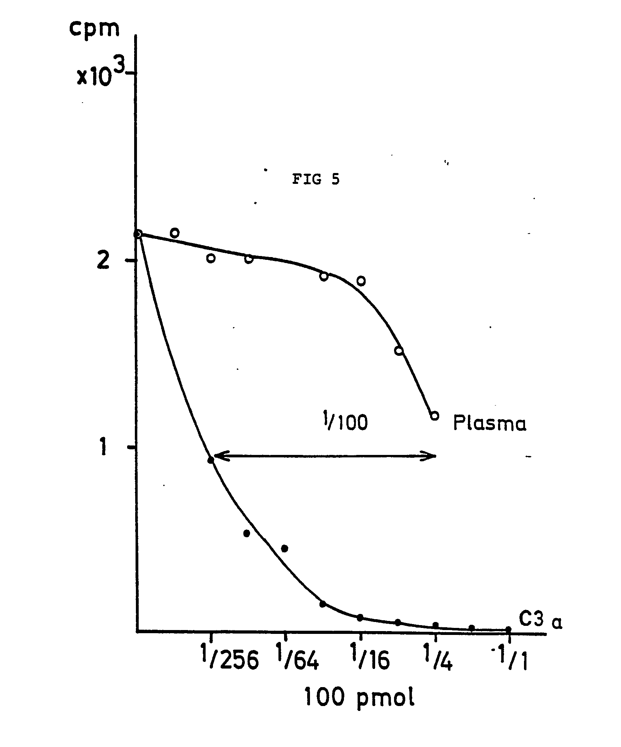

**Claims**

1. Anti-C3a antibody preparation, characterized in that in its reaction with complement fragment C3a it is substantially not inhibited by complement factor C3 so that it can be employed for C3a assays, for example in plasma samples, in the presence of native C3.

2. Anti-C3a antibody preparation according to claim 1, characterized by being directed specifically against at least one antigenic determinant in C3a which is not accessible in the C3a region of native C3.

3. Anti-C3a antibody preparation according to claim 2, characterized in that the antigenic determinant(s) against which it is directed in C3 is/are exposed when C3 has been denatured in an aqueous solution containing SDS $10^{-3}$M, at a pH of about 7 and room temperature.

4. Anti-C3a antibody preparation according to any of claims 1—3, characterized by having been produced by means of immunization with an immunogen which carries at least one of the said determinants and which is selected from among (i) denatured C3, especially SDS denatured, (ii) denatured fragment of C3 which contains the C3a region, and (iii) C3a, whereupon the immune response so obtained is limited by selecting these antibodies that are directed specifically against a determinant in C3a which is not accessible in the C3a-region of native C3.

5. Method for determining the complement fragment C3a by immunochemical assay, characterized in that the anti-C3a antibody preparation employed is substantially uninhibited by complement factor C3 in its reaction with C3a.

6. Method for determining C3a by immunochemical assay, characterized in that the anti-C3a antibody preparation employed is directed specifically against at least one such determinant in C3a that is not accessible in the C3a region of native C3 but is exposed upon denaturation of C3.

7. Method according to claim 6, characterized in that the C3a-region determinant(s) against which the preparation employed is directed is/are exposed upon denaturation with SDS $10^{-3}$M at a pH of about 7 and room temperature.

8. Method according to either one of claims 6 and 7, characterized in that the anti-C3a antibody preparation employed has been produced a) by means of immunization with an immunogen which carries at least one of the said determinants and which is selected from among (i) denatured C3, especially SDs denatured, (ii) denatured fragment of C3 which contains the C3a region, and (iii) C3a, followed b) by selection of such antibodies of the immune response so obtained that are directed specifically against a determinant in C3a which is not accessible in the C3a-region of native C3.

**Patentansprüche**

1. Anti-C3a-Antikörperzubereitung, dadurch gekennzeichnet, daß sie in ihrer Reaktion mit dem Komplimentfragment C3a im wesentlichen nicht durch den Komplementfaktor C3 inhibiert wird, so daß sie zur Analyse auf C3a z.B. in Plasmaproben in Gegenwart von nativem C3 verwendet werden kann.

2. Anti-C3a-Antikörperzubereitung nach Anspruch 1, dadurch gekennzeichnet, daß sie spezifisch gegen mindestens eine antigene Determinante von C3a, die in der C3a-Region von nativem C3 nicht zugänglich ist, gerichtet ist.

3. Anti-C3a-Antikörperzubereitung nach Anspruch 2, dadurch gekennzeichnet, daß die antigene Determinante bzw. Determinanten, gegen die sie in C3 gerichtet ist, exponiert wird bzw. werden, wenn C3 in einer wäßrigen Lösung, die $10^{-3}$M SDS enthält, bei einem pH von etwa 7 und Raumtemperatur denaturiert wurde.

4. Anti-C3a-Antikörperzubereitung nach irgendeinem der Ansprüche 1 bis 3, dadurch gekennzeichnet, daß sie durch Immunisierung mit einem Immunogen, das mindestens eine der genannten Determinanten trägt und das ausgewählt ist aus (i) denaturiertem C3, insbesondere SDS-denaturiertem, (ii) denaturiertem Fragment von C3, das die C3a-Region enthält, und (iii) C3a hergestellt wurde, worauf die so erhaltene Immunantwort durch Selektion der Antikörper, die spezifisch gegen eine Determinante von C3a, die in der C3a-Region von nativem C3 nicht zugänglich ist, gerichtet sind, beschränkt wird.

5. Verfahren zur Bestimmung des Komplementfragments C3a mittels immunochemischer Analyse, dadurch gekennzeichnet, daß die verwendete Anti-C3a-Antikörperzubereitung im wesentlichen nicht durch den Komplementfactor C3 in seiner Reaktion mit C3a inhibiert wird.

6. Verfahren zur Bestimmung von C3a mittels immunochemischer Analyse, dadurch gekennzeichnet, daß die verwendete Anti-C3a-Antikörperzubereitung spezifisch gegen mindestens eine solche Determinante von C3a gerichtet ist, die in der C3a-Region von nativem C3 nicht zugänglich ist, aber nach Denaturierung von C3 freigelegt wird.

7. Verfahren nach Anspruch 6, dadurch gekennzeichnet, daß die Determinante bzw. Determinanten der C3a-Region, gegen die die verwendete Zubereitung gerichtet ist, nach Denaturierung mit $10^{-3}$M SDs bei einem pH von etwa 7 und Raumtemperatur freigelegt wird bzw. werden.

8. Verfahren nach Anspruch 6 oder 7, dadurch gekennzeichnet, daß die verwendete Anti-C3a-Antikörperzubereitung hergestellt wurde durch a) Immunisierung mit einem Immunogen, das mindestens eine der genannten Determinanten trägt und das ausgewählt ist aus (i) denaturiertem C3, insbesondere SDS-denaturiertem, (ii) denaturiertem Fragment aus C3, das die C3a-Region enthält, und (iii) C3a und anschließend durch b) Selektion solcher Antikörper der so erhaltenen Immunantwort, die spezifisch gegen eine Determinante auf C3a, die in der C3a-Region von nativem C3 nicht zugänglich ist, gerichtet sind.

**Revendications**

1. Préparation d'anticorps anti-C3a, caractérisée en ce que dans sa réaction avec le fragment du complément C3a, elle n'est pratiquement pas inhibée par le facteur du complément C3 si bien qu'elle peut être employée pour des essais de C3a, par exemple dans des échantillons de plasma, en présence de C3 naturel.

2. Préparation d'anticorps anti-C3a suivant la revendication 1, caractérisée en ce qu'elle est dirigée spécifiquement contre au moins un déterminant antigénique dans C3a qui n'est pas accessible dans la région C3a du C3 naturel.

3. Préparation d'anticorps anti-C3a suivant la revendication 2, caractérisée en ce que le ou les déterminants antigéniques contre lesquels elle est dirigée dans C3 sont exposés lorsque C3 a été dénaturé dans une solution aqueuse contenant du SDS $10^{-3}$M, à un pH d'environ 7 et à la température ambiante.

7

4. Préparation d'anticorps anti-C3a suivant l'une quelconque des revendications 1 à 3, caractérisée en ce qu'elle a été produite par immunisation avec un immunogène qui porte au moins l'un de ces déterminants et qui est choisi parmi (i) du C3 dénaturé, en particulier dénaturé par SDS, (ii) un fragment dénaturé de C3 qui contient la région C3a et (iii) du C3a, après quoi la réponse immune ainsi obtenue est limitée par le choix de ces anticorps qui sont dirigés spécifiquement contre un déterminant dans C3a qui n'est pas accessible dans la région C3a du C3 naturel.

5. Procédé pour déterminer le fragment du complément C3a par essai immunochimique, caractérisé en ce que la préparation d'anticorps anti-C3a employée est pratiquement non inhibée par le facteur du complément C3 dans sa réaction avec C3a.

6. Procédé pour déterminer C3a par essai immunochimique, caractérisé en ce que la préparation d'anticorps anti-C3a employée est dirigée spécifiquement contre au moins un déterminant dans C3a qui n'est pas accessible dans la région C3a du C3 naturel, mais est exposé après dénaturation de C3.

7. Procédé suivant la revendication 6, caractérisé en ce que le ou les déterminants antigéniques de la région C3a contre lesquels la préparation employée est dirigée, sont exposés après dénaturation avec du SDS $10^{-3}$M, à un pH d'environ 7 et à la température ambiante.

8. Procédé suivant l'une quelconque des revendications 6 ou 7, caractérisé en ce que la préparation employée a été produite a) par immunisation avec un immunogène qui porte au moins l'un de ces déterminants et qui est choisi parmi (i) du C3 dénaturé, en particulier dénaturé par SDS, (ii) un fragment dénaturé de C3 qui contient la région C3a et (iii) du C3a, puis b) par sélection de ces anticorps de la réponse immune ainsi obtenue qui sont dirigés spécifiquement contre un déterminant dans C3a qui n'est pas accessible dans la région C3a du C3 naturel.

EP 0 241 443 B1

FIG 1

FIG 2

FIG 3

FIG 4

FIG 5